# FASCICULE DE BREVET EUROPEEN

(11) **EP 1 885 279 B1**
(45) Date de publication et mention de la délivrance du brevet: **07.01.2015**
(21) Numéro de dépôt: 06743699.8
(22) Date de dépôt: 14.04.2006
(51) Int. Cl.: A61F 2/00

(54) **ENSEMBLE POUR LE TRAITEMENT CHIRURGICAL D'UN PROLAPSUS ET IMPLANT ADAPTE POUR UN TEL TRAITEMENT**
SYSTEM ZUR CHIRURGISCHEN BEHANDLUNG EINES PROLAPSUS UND EIN ZUR BEHANDLUNG GEEIGNETES IMPLANTAT
ARRANGEMENT FOR SURGICALLY TREATING A PROLAPSE AND AN IMPLANT FOR SAID TREATMENT

(30) Priorité: 15.04.2005 FR 0503797
(43) Date de publication de la demande: 13.02.2008
(73) Titulaire: SOFRADIM PRODUCTION, 01600 Trévoux (FR)
(72) Inventeur: THERIN, Michel, 69004 Lyon (FR); SPINNLER, Linda, 69480 POMMIERS (FR)
(74) Mandataire: Brédeville, Odile Marie
(86) Numéro de dépôt international: PCT/FR2006/000826
(87) Numéro de publication internationale: WO 2006/108964

(56) Documents cités:
- WO-A-03/096929
- US-A1- 2003 009 181
- US-A1- 2004 015 048

## Description

La présente invention concerne le traitement chirurgical d'un prolapsus affectant un organe ou une partie d'organe protrudant à savoir le rectum, ou les organes génitaux chez la femme, dont l'utérus et vagin, ou la vessie.

La présente invention concerne en particulier le traitement des prolapsus des voies génitales antérieure et postérieure chez la femme, dont cystocèle et rectocèle.

Comme montré au haut à la figure 1 du document FR-A-2859624, on connaît déjà un implant pour un traitement chirurgical tel que défini précédemment, ayant la forme d'une pièce, par exemple textile, mince et conformable, et comprenant :
- une base de support ou soutien de l'organe protrudant, s'étendant d'un bord antérieur à un bord postérieur, avec le cas échéant deux bords latéraux entre lesdits bords antérieur et postérieur
- au moins quatre bras de suspension, dont deux antérieurs en vis-à-vis, et deux postérieurs en vis à vis, s'étendant chacun à partir de la base de support vers une extrémité libre d'ancrage dans une structure anatomique stable au voisinage de l'organe protrudant.
Un tel implant peut appartenir à un ensemble ou kit pour le traitement chirurgical du prolapsus, comprenant en outre un ancillaire de perforation guidée de la région anatomique à disséquer, voisine de l'organe protrudant. Cet ancillaire comprend une tige recourbée dont la partie distale comporte, et une extrémité pointue permettant de créer un passage dans la région anatomique précitée, et un moyen de liaison amovible avec une extrémité libre d'ancrage de l'implant, ainsi qu'une poignée solidaire de la partie proximale de la tige.

La présente invention a pour objet un ensemble pour le traitement chirurgical d'un prolapsus, particulièrement simple d'utilisation pour le chirurgien, car ne requérant pas en particulier de connexion complexe entre l'implant et les autres composant du kit, dont l'ancillaire de perforation guidée.

Un autre objet de la présente invention est un kit ou ensemble pour traitement chirurgical, permettant d'exposer l'implant le plus tard possible au milieu in vivo septique.

Un autre objet de l'invention est un implant tel que précédemment défini, comportant un moyen de protection provisoire, vis-à-vis du milieu in vivo septique.

Conformément à la présente invention, un ensemble pour le traitement chirurgical d'un prolapsus, comprenant un implant et un ancillaire de perforation guidée tels que précédemment définis, se caractérise par le fait que, en combinaison et en coopération, d'une part, au moins une extrémité libre d'ancrage de l'implant comporte un élément de traction, par exemple une boucle, et d'autre part la partie distale de l'ancillaire de perforation comporte un élément de préhension amovible, par exemple un crochet, d'un élément de traction de l'implant.

Conformément à la présente invention, l'implant comprend des moyens de protection temporaire, comprenant une pochette constituée par au moins une paroi isolante par rapport à la région anatomique à disséquer. Cette pochette est agencée pour recevoir la base de support de l'implant, et les bras de suspension en position repliée sur la base de support, mais avec les éléments de traction traversant la paroi de la pochette, et demeurant à l'extérieur de cette dernière.

Ces moyens particuliers permettent une exposition très courte de l'implant, à la fin de l'intervention chirurgicale, et donc une limitation des risques d'infection.

Préférentiellement, ce même ensemble comprend une baguette de traction intermédiaire, comportant à une extrémité un élément de traction, par exemple une boucle, et à son autre extrémité, un élément de préhension amovible, par exemple un crochet, la baguette ayant une surface extérieure lisse pour un passage non traumatisant dans la région anatomique à disséquer, perforée avec l'ancillaire de perforation guidée.

Comme décrit ci-après, cette baguette de traction intermédiaire s'avère particulièrement avantageuse, en ce qu'elle permet de réserver un passage dans les tissus et/ou autres structures anatomiques au voisinage de l'organe protrudant, et d'exposer l'implant à ceux-ci le plus tard possible. L'utilisation de cette ou de ces baguettes de traction intermédiaire permet d'éviter d'avoir l'implant dans le champ opératoire dès que le premier bras de suspension se trouve engagé dans un passage, vers une structure anatomique stable au voisinage de l'organe protrudant.

La présente invention est maintenant décrite par rapport au dessin annexé, dans laquelle :
- la figure 1 représente, de manière schématique un kit ou ensemble pour le traitement chirurgical d'un prolapsus, selon la présente invention,
- la figure 2 représente, en vue de dessus, un implant appartenant au kit selon l'invention, et pouvant être appréhendé indépendamment de ce dernier,
- la figure 3 est une vue de côté de la partie distale d'un ancillaire de perforation guidée, appartenant au kit ou ensemble selon l'invention,
- la figure 4 illustre le mode de connexion ou préhension amovible entre, d'un côté l'élément de préhension, ou crochet, appartenant à l'ancillaire de perforation d'un kit selon l'invention, et de l'autre côté un élément de traction, par exemple une boucle, appartenant à une extrémité libre d'ancrage d'un implant,
- la figure 5 illustre le mode de connexion ou préhension amovible entre, d'un côté l'élément de préhension, ou crochet, appartenant à une baguette de traction intermédiaire d'un kit selon l'invention, et de l'autre coté un élément de traction, par exemple une boucle, appartenant à une extrémité libre d'ancrage d'un implant,
- la figure 6 illustre un implant représenté à la figure 2, mais associé à des moyens de protection temporaire, les bras de suspension étant en voie de dépliement sous l'effet d'une traction exercée à leurs extrémités libres d'ancrage.

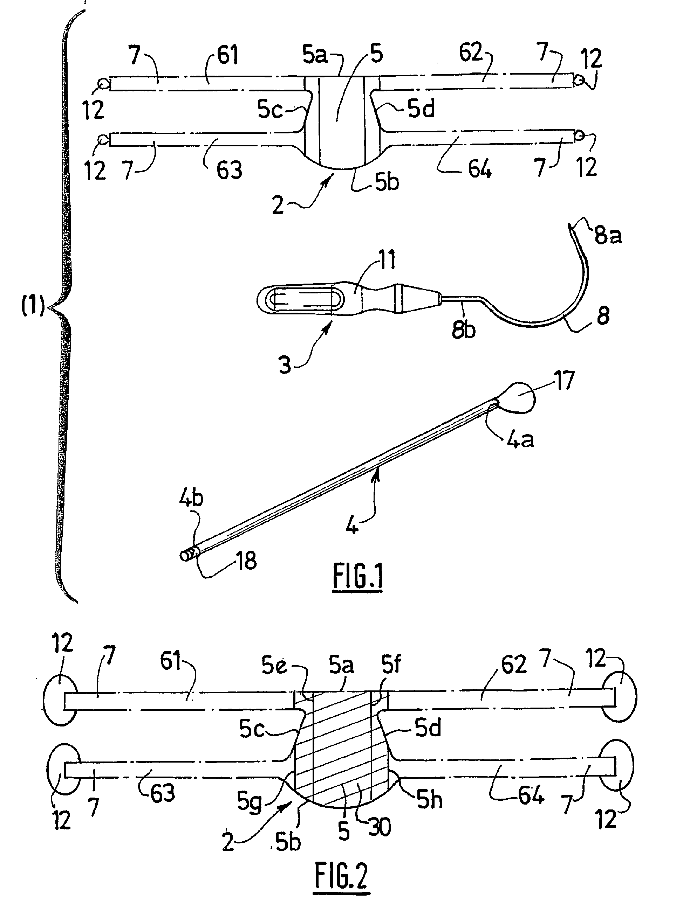

Comme montré à la figure 1, l'ensemble 1 ou kit pour le traitement chirurgical d'un prolapsus comprend :
- un implant 2 ayant la forme d'une pièce mince et conformable
- un ancillaire 3 de perforation guidée de la région anatomique à disséquer
- et une baguette 4 de traction intermédiaire.

L'implant 2 comprend de manière monobloc ou en une seule pièce :
- une base 5 de support ou soutien de l'organe protrudant, s'étendant d'un bord antérieur 5a à un bord postérieur 5b, avec le cas échéant deux bords latéraux 5c et 5d entre lesdits bords antérieur et postérieur,
- au moins quatre bras de suspension, dont deux antérieurs 61 et 62 en vis-à-vis, et deux postérieurs 63 et 64 en vis-à-vis, s'étendant chacun à partir de la base de support 5 vers une extrémité libre d'ancrage 7 dans une structure anatomique stable au voisinage de l'organe protrudant.

La base de support 5 a une forme générale rectangulaire ou carrée, avec un bord antérieur 5a droit, un bord postérieur 5b convexe, et deux bord latéraux 5c et 5d s'étendant obliquement vers le bord postérieur 5b. Ce dernier en position rabattue peut être positionné sur le col utérin en cas de conservation utérine, ou positionné au dessus du dôme vaginal, lorsque l'utérus est retiré.

Les bras de suspension 61 à 64 s'étendent parallèlement les uns aux autres, deux à deux ; autrement dit un bras antérieur à 61 ou 62 s'étend parallèlement à un bras postérieur 63 ou 64, et ce dans une direction sensiblement perpendiculaire au plan de symétrie de l'implant 2.

Il doit être entendu que, d'une part le nombre de bras de suspension peut être supérieur à quatre, et d'autre part leur orientation relative, deux à deux, peut être différente de celles représentées à la figure 2, en fonction en particulier du prolapsus traité chirurgicalement. Par exemple, au lieu d'être alignés, les bras 61 et 62 peuvent définir entre eux un angle obtus ou autre, et de même pour les bras postérieurs 63 et 64, lesquels peuvent définir entre eux un angle différent de celui existant entre les bras antérieurs 61 et 62.

Chacune des extrémités libres 7 d'ancrage comporte un élément de traction consistant en une boucle 12 dans un matériau polymère bio compatible. Lorsque chacun des bras 61 à 64 est obtenu, comme décrit ci-après, à partir d'un tricot prothétique en matériau polymère biocompatible, chacune des boucles 12 est fixée à une extrémité 7 d'ancrage, de manière libre, par repli de ladite extrémité sur elle-même, et soudure par ultra-sons.

Ce mode particulier est atraumatique, crée un encombrement minimum pour le passage anatomique, tout en présentant de bonnes propriétés mécaniques, équivalentes à celles des connecteurs connus aujourd'hui aux mêmes fins.

En termes de réalisation, l'implant 2 consiste tout d'abord en une pièce mince et conformable, obtenue à partir d'un tricot prothétique ajouré fait d'une seule pièce. Le tricot consiste en un arrangement constitué par plusieurs nappes de fils d'un matériau polymère biocompatible, par exemple polypropylène, comprenant, de manière continue dans le sens du tricot, la base 5 de support, et les quatre bras 61 à 62 de suspension de part et d'autres de la dite base.

En utilisant les modes de tricotage décrits dans le document FR-A-2859624, le tricot prothétique a des propriétés ou caractéristiques différenciées selon la partie ou zone de l'implant 2 considérée. La masse surfacique du tricot dans la base 5 de support est supérieure à la masse surfacique du tricot dans chacun des bras 61 à 64 de suspension. Et l'élasticité du tricot dans la base de support 5 est supérieure à l'élasticité du tricot dans chacun des bras de suspension 61 à 64. Ces caractéristiques mécaniques différenciées peuvent être obtenues en particulier avec un tricot prothétique comprenant :
- au moins deux nappes de base, à savoir une nappe avant et une nappe arrière, s'étendant sur la surface de la base 5 de support et les quatre bras 61 à 64, les deux dites nappes de base étant maillantes et définissant une première armure,
- au moins deux nappes supplémentaires, en arrière de la dite nappe de base arrière, s'étendant principalement sur les surfaces respectives des bras de suspension 61 à 64, ces deux nappes supplémentaires étant des nappes non maillantes de trame partielle et définissant une deuxième armure. A cet égard, on se référera en particulier au document FR-A-2859624.

A titre d'exemple, les deux nappes supplémentaires définissent deux lignes 5e et 5f, séparant d'un côté, c'est-à-dire du côté des bras, l'extension desdites nappes supplémentaires, et de l'autre côté, c'est-à-dire entre les deux lignes 5e et 5f, la partie du tricot constitué uniquement par les deux nappes de base.

Le tricot prothétique ajouré ainsi obtenu ou construit constitue un support textile poreux comprenant un arrangement de fils composés chacun d'au moins un brin en matériau polymère, s'étendant sur et correspondant à l'ensemble constitué, en une seule pièce, par la base 5 de support et les bras 61 à 64 de suspension.

L'implant représenté selon la figure 2 est en fait une pièce composite, en ce sens qu'il comprend, outre le support textile poreux précédemment défini, un matériau résorbable hydrophile 20 revêtant principalement la base 5 de support, par exemple entre les lignes 5g et 5h représentées à la figure 2. Par "matériau résorbable hydrophile", on entend et on comprend tout matériau susceptible d'être dégradé et éliminé in vivo, tel que collagène ou polysaccharides.

Ce matériau résorbable hydrophile peut revêtir la base 5 de support de différentes manières. Tout d'abord il peut consister en une ou deux couches planes, ou membranes, revêtant respectivement une ou deux faces du support textile de la base 5. Mais, selon un mode préféré d'exécution de la présente invention, le matériau résorbable hydrophile enduit le support textile poreux, en formant un film enveloppant et pénétrant dans l'arrangement de fils du support textile poreux, en obturant au moins sa texture microporeuse, c'est-à-dire celle comprenant les interstices compris entre au moins deux fils aux endroits de contact d'un fil avec au moins un autre fil, et ce dans la base de support 5, mais sans former une couche plane revêtant au moins une face du support textile. A cet égard, on met en oeuvre la technique d'enduction décrite par le document WO 2004/043294.

Compte tenu de la nature textile ou tricotée du support textile poreux précédemment défini, il doit être entendu que l'élément de traction à chaque extrémité libre 7 d'ancrage de l'implant peut consister en un croisement ou maille, identifié ou non, des fils constitutifs du support poreux, se trouvant accroché de manière amovible, soit par l'élément de préhension amovible de l'ancillaire 3 de perforation, soit par l'élément de traction d'une baguette 4 de traction intermédiaire. En pareil cas, il n'y a donc pas de boucle telle que précédemment décrite.

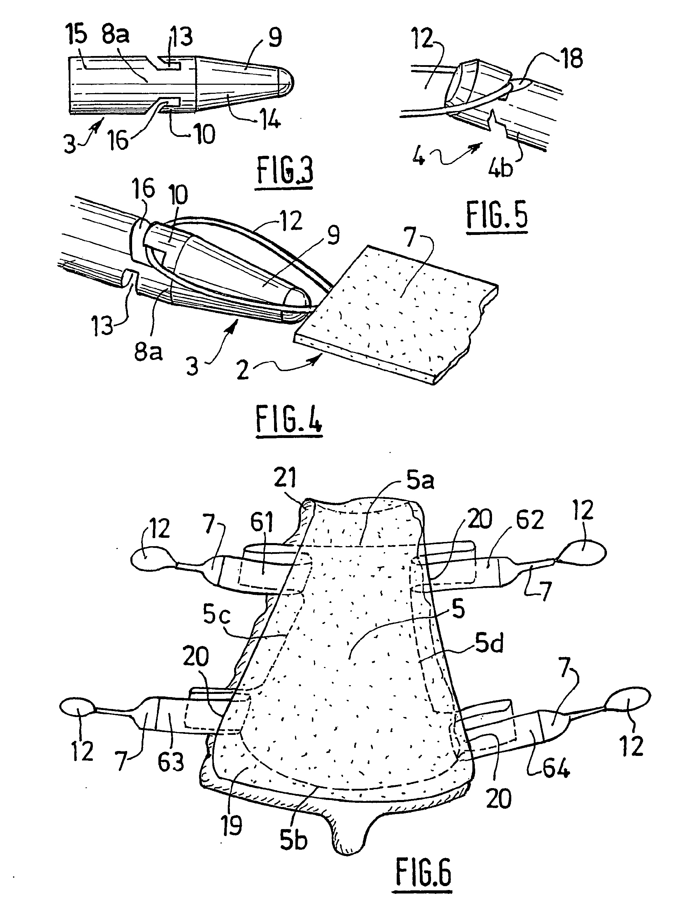

Comme représenté plus particulièrement à la figure 6, l'implant 2 est associé ou comprend des moyens de protection temporaire par rapport à la région anatomique à disséquer, septique. Ces moyens de protection temporaire comprennent une pochette 19 constituée par au moins une paroi ou matière isolante, ou étanche, par rapport à la région anatomique à disséquer. La pochette a une forme et des dimensions généralement adaptées à la réception de l'implant 2 précédemment décrit. Plus précisément la pochette 19 est agencée pour recevoir la base 5 de support et les bras de suspension 61 à 64, en position repliée sur la base 5, mais avec au moins un, sinon la totalité des éléments de traction 12 traversant la paroi de la pochette et demeurant à l'extérieur de cette dernière. Même si l'implant 2 se trouve contenu et emballé dans la pochette 19, il demeure néanmoins possible de saisir les bras de suspension par l'intermédiaire des éléments de traction ou boucles 12.

La paroi de la pochette 19 est faite en un matériau ou film thermoplastique déchirable et/ou sécable. Par conséquent, toute traction d'un bras de suspension 61 à 64 se dépliant, à partir des ouvertures 20 générées ou préexistant dans la pochette 19, élargit ces dernières, et permet ensuite au chirurgien de séparer la pochette en plusieurs morceaux extraits du champ opératoire.

La pochette comporte par ailleurs un rabat de fermeture 21 au niveau du bord antérieur 5a de la base 5 de l'implant 2.

En pratique, la pochette 19 est obtenue à partir de deux volets découpés dans un film thermoplastique, rabattus l'un sur l'autre, au-dessus de l'implant 2, et les bords jointifs des deux rabats sont thermo-soudés par ultrasons.

Comme montré par les figures 1, 3 et 4, l'ancillaire 3 de perforation guidée comprend une tige 8 recourbée, ayant toute forme adaptée aux gestes opératoires, dont la partie distale 8a comporte, et une extrémité pointue 9, et un moyen 10 de liaison amovible avec une extrémité libre 7 d'ancrage de l'implant, ainsi q'une poignée 11 solidaire de la partie proximale de la tige 8. Le moyen 10 de liaison amovible comporte un élément de préhension amovible, par exemple un crochet 13, d'un élément de traction 12, ou crochet, appartenant soit à une extrémité 7 d'ancrage de l'implant 2, soit à la baguette 4 de traction intermédiaire, décrite ci-après.

Plus particulièrement, comme montré par les figures 3 et 4, la partie distale 8 a de l'ancillaire 3 de perforation guidée comporte une partie effilée 14 prolongée vers la poignée 11 par une partie pleine 15, dans laquelle est ménagée au moins une encoche 16 de pénétration et retenue d'un élément de traction 12 ou 17, soit de l'implant 2, soit de la baguette de traction intermédiaire 4.

Comme montré par la figure 1, la baguette 4 de traction intermédiaire, consistant par exemple en une tige ou un tube en matière plastique flexible, comporte, à une extrémité 4a un élément de traction, par exemple une boucle 17, identique ou analogue à un élément de traction 12 de l'implant 2, et à son autre extrémité 4b un élément de préhension amovible, par exemple un crochet 18, analogue ou identique à celui de l'ancillaire 3 de perforation. La baguette 4 a une surface extérieure lisse pour un passage non traumatisant dans la région anatomique à disséquer, perforée avec l'ancillaire 3.

Le kit précédemment décrit peut être mis en oeuvre avec ou sans les baguettes 4 de traction intermédiaire, à partir du moment où le chirurgien a effectué un premier passage avec l'ancillaire 3 de perforation à travers la région anatomique à disséquer.

Avec une baguette 4 de traction intermédiaire, le chirurgien accroche la boucle 17 de cette dernière sur le crochet 13 de l'ancillaire 3. En retirant ce dernier, il place la baguette 4 dans le trajet. Puis il décroche la boucle 17 du crochet 13 de l'ancillaire, et laisse la baguette 4 en place dans le trajet. Puis le chirurgien recommence cette manoeuvre avec les trois autres baguettes 4 de traction intermédiaire, respectivement pour les trois autres trajets requis pour la mise en place et fixation de l'implant 2.

Les baguettes étant ainsi mises en place, l'implant 2 est alors utilisé. Pour mettre en place ce dernier, le chirurgien n'a plus qu'à accrocher chaque boucle 12 sur le crochet 18 de chaque baguette 4, et à tirer sur les dites baguettes afin de placer les bras de suspension 61 à 64 en différents passages. En tirant ainsi sur chaque bras 61 à 64, les soudures de la pochette 19 se cassent, et cette dernière peut alors être retirée à la fin de l'intervention chirurgicale. Le chirurgien peut alors faire quelques points de suture pour fixer l'implant 2 s'il le souhaite.

Sans utiliser les baguettes 4 de traction intermédiaire, le chirurgien connecte alors directement un crochet 13 de l'ancillaire à une boucle 12 de l'implant 2. En retirant l'ancillaire 3, chaque bras 61 à 64 de l'implant est placé dans un passage, et comme précédemment, les soudures de la pochette 19 se cassent.

En recommençant cette même procédure pour chacun des bras 61 à 64 de l'implant 2, et en retirant la pochette 19, on aboutit à la mise en place de l'implant 2 dans le site retenu par le chirurgien. Comme précédemment, le chirurgien peut ensuite faire quelques points de sutures pour fixer l'implant, si cela est requis.

La présente invention concerne aussi un procédé de traitement chirurgical d'un prolapsus affectant un organe ou une partie d'organe protrudant, choisi dans le groupe constitué par le rectum, les organes génitaux chez la femme, dont utérus et vagin, et la vessie, mettant en oeuvre un ensemble tel que décrit par référence à l'ensemble des figures, caractérisé en ce que
(a) éventuellement, le chirurgien effectue un passage initial à l'ancillaire (3) de perforation dans la région anatomique à disséquer, voisine de l'organe protrudant
(b) le chirurgien lie l'élément de traction (17) de la baguette (4) intermédiaire de traction avec l'élément de préhension (7) de l'ancillaire de perforation, puis il effectue un passage dudit ancillaire dans ladite région anatomique à disséquer ; et, en retirant l'ancillaire, il laisse la baguette intermédiaire de traction dans le trajet ménagé avec ledit ancillaire
(c) le chirurgien lie l'élément de traction (7) d'un bras de suspension (61 64) de l'implant (2) avec l'élément de préhension (18) d'une baguette intermédiaire (4), puis en tirant sur l'élément de traction (17) de ladite baguette, introduit et place ledit bras de suspension dans ledit trajet
(d) le chirurgien fixe l'extrémité libre (12) d'ancrage dudit bras sur une structure anatomique stable au voisinage de l'organe protrudant.

Le chirurgien répète les opérations (a) à (e) pour les quatre bras de suspension (61 à 64) respectivement.

## Revendications

1. Ensemble (1) pour le traitement chirurgical d'un prolapsus affectant un organe ou une partie d'organe protrudant, choisi dans le groupe constitué par le rectum, les organes génitaux chez la femme, dont utérus et vagin, et la vessie, comprenant :
1.1) un implant (2) ayant la forme d'une pièce mince et conformable, et comprenant :
- une base (5) de support ou soutien de l'organe protrudant, s'étendant d'un bord antérieur (5a) à un bord postérieur (5b), avec éventuellement deux bords latéraux (5c) (5d) entre lesdits bords antérieur et postérieur,
- au moins quatre bras de suspension, dont deux antérieurs (61, 62) en vis-à-vis, et deux postérieurs (63, 64) en vis-à-vis, s'étendant chacun à partir de la base de support vers une extrémité libre d'ancrage (7) dans une structure anatomique stable au voisinage de l'organe protrudant
1.2) un ancillaire (3) de perforation guidée de la région anatomique à disséquer, voisine de l'organe protrudant, comprenant une tige (8) recourbée dont la partie distale (8a) comporte, et une extrémité pointue (9), et un moyen (10) de liaison amovible avec une extrémité libre (7) d'ancrage de l'implant (2), ainsi qu'une poignée (11) solidaire de la partie proximale de la tige (8), d'une part au moins une extrémité libre (7) d'ancrage comporte un élément de traction, par exemple une boucle (12), et d'autre part la partie distale (8a) de l'ancillaire de perforation comporte un élément de préhension amovible, par exemple un crochet (13), de l'élément de traction (12),
**caractérisé en ce que** l'implant (2) comprend des moyens de protection temporaire par rapport à la région anatomique à disséquer, septique, les moyens de protection temporaire comprenant une pochette (19) constituée par au moins une paroi isolante par rapport à la région anatomique à disséquer, ladite pochette étant agencée pour recevoir la base (5) de support de l'implant et les bras de suspension (61 à 64) en position repliée sur la base de support, mais avec au moins un élément de traction (12) traversant la paroi et demeurant à l'extérieur de la pochette (19).

2. Ensemble selon la revendication 1, **caractérisé en ce que** la partie distale (8a) de l'ancillaire (3) de perforation guidée comporte une partie effilée (14) prolongée vers la poignée (11) par une partie pleine (15) dans laquelle est ménagée au moins une encoche (16) de pénétration et retenue d'un élément de traction (12) de l'implant (2).

3. Ensemble selon la revendication 1, **caractérisé en ce qu'**il comprend au moins une baguette (4) de traction intermédiaire, comportant, à une extrémité (4a) un élément de traction, par exemple une boucle (17), et à son autre extrémité (4b) un élément de préhension amovible, par exemple un crochet (18), la baguette ayant une surface extérieure lisse pour un passage non traumatisant dans la région anatomique à disséquer, perforée avec l'ancillaire (3) de perforation guidée.

4. Ensemble selon la revendication 1, **caractérisé en ce que** la paroi est faite en un matériau thermo-plastique déchirable et/ou sécable, à partir des ouvertures (20) générées dans la pochette (19) par la traction d'un bras de suspension (61 à 64) se dépliant, et élargissant lesdites ouvertures.

5. Ensemble selon la revendication 1, **caractérisé en ce que** la pochette comporte un rabat de fermeture (21) au niveau du bord antérieur (5a) de la base (5) de l'implant.

6. Implant (2) pour le traitement chirurgical d'un prolapsus affectant un organe ou une partie d'organe protrudant choisi dans le groupe constitué par le rectum, les organes génitaux chez la femme, dont l'utérus et vagin, et la vessie, ledit implant ayant la forme d'une pièce mince et conformable, et comprenant :
- une base (5) de support ou soutien de l'organe protrudant, s'étendant d'un bord antérieur (5a) à un bord postérieur (5b) avec éventuellement deux bords latéraux (5c, 5d) entre lesdits bords antérieur et postérieur,
- au moins quatre bras (61 à 64) de suspension, dont deux antérieurs (61, 62) en vis-à-vis, et deux postérieurs (63, 64) en vis-à-vis, s'étendant chacun à partir de la base de support (5) vers une extrémité libre d'ancrage (7) dans une structure anatomique stable au voisinage de l'organe protrudant,
- au moins une extrémité libre (7) d'ancrage comportant un élément de traction, par exemple une boucle (12),
**caractérisé en ce qu'**il comprend des moyens de protection temporaire, ces derniers comprenant une pochette (19) constituée par au moins une paroi isolante par rapport à la région anatomique à disséquer, ladite pochette étant agencée pour recevoir la base (5) de support de l'implant et les bras de suspension (61 à 64) en position repliée sur la base de support, mais avec au moins un élément de traction (12) traversant la paroi et demeurant à l'extérieur de la pochette (19).

7. Implant selon la revendication 6, **caractérisé en ce que** les quatre extrémités libres (7) d'ancrage comportent respectivement quatre éléments de traction (12).

8. Implant selon la revendication 6, **caractérisé en ce que** la paroi est faite en un matériau thermo-plastique déchirable et/ou sécable, à partir des ouvertures (20) générées dans le sachet (19) par la traction d'un bras de suspension (61 à 64) se dépliant, et élargissant lesdites ouvertures.

9. Implant selon la revendication 6, **caractérisé en ce que** la pochette comporte un rabat de fermeture (21) au niveau du bord antérieur (5a) de la base (5) de l'implant.

10. Implant selon la revendication 6, **caractérisé en ce que** la pièce mince et conformable comprend un tricot prothétique ajouré fait d'une seule pièce, à base d'un arrangement constitué par plusieurs nappes de fils d'un matériau polymère biocompatible, comprenant, de manière continue dans le sens du tricot, la base (5) de support, et les quatre bras (61à 64) de suspension de part et d'autre de la base (5) de support, et l'élasticité du tricot dans la base de support étant supérieure à l'élasticité du tricot dans chacun des bras de suspension.

11. Implant selon la revendication 6, **caractérisé en ce que** la masse surfacique du tricot dans la base (5) de support est supérieure à la masse surfacique du tricot dans chacun des bras (61 à 64) de suspension.

12. Implant selon la revendication 10 ou 11, **caractérisé en ce que** le tricot prothétique comprend :
- au moins deux nappes de base, une nappe avant et une nappe arrière, s'étendant sur la surface de la base (5) de support et les quatre bras (61 à 64), les deux dites nappes de base étant maillantes et définissant une première armure,
- au moins deux nappes supplémentaires, en arrière de la dite nappe de base arrière, s'étendant principalement sur les surfaces respectives des bras de suspension (61 à 64), les deux dites nappes supplémentaires étant des nappes non maillantes de trame partielle et définissant une deuxième armure.

13. Implant selon la revendication 6, **caractérisé en ce que** la pièce mince et conformable est composite, et comprend :
- un support textile poreux comprenant un arrangement de fils composés chacun d'au moins un brin en matériau polymère, ledit support textile s'étendant sur et correspondant à la base (5) de support et aux bras (61 à 64) de suspension,
- un matériau résorbable hydrophile (30) revêtant principalement la base (5) de support.

14. Implant selon la revendication 13, **caractérisé en ce que** le support textile poreux définit une texture microporeuse comprenant les interstices compris entre au moins deux fils aux endroits de contact d'un fil avec au moins un autre fil, et, dans la base de support (5), le matériau résorbable hydrophile enduit le support textile, en formant un film enveloppant et pénétrant dans l'arrangement de fils, en obturant au moins la texture microporeuse, mais sans former une couche plane revêtant au moins une face de support textile.

## Patentansprüche

1. Einheit (1) zur chirurgischen Behandlung eines Prolapses, der ein prolabierendes Organ oder einen Abschnitt eines prolabierenden Organs betrifft, das aus der Gruppe bestehend aus dem Rektum, den weiblichen Genitalorganen, darunter der Uterus und die Vagina, und der Blase ausgewählt ist, wobei die Einheit Folgendes umfasst:
1.1) ein Implantat (2), das die Form eines dünnen und anpassbaren Teils hat und Folgendes umfasst:
- eine Basis (5) zum Tragen oder Stützen des prolabierenden Organs, die sich von einer Vorderkante (5a) zu einer Hinterkante (5b) erstreckt, mit gegebenenfalls zwei Seitenkanten (5c), (5d) zwischen der besagten Vorderkante und der besagten Hinterkante,
- mindestens vier Aufhängungsarme, darunter zwei gegenüberliegende vordere (61, 62) und zwei gegenüberliegende hintere (63, 64), die sich jeweils von der Tragbasis aus zu einem freien Verankerungsende (7) hin in einer stabilen anatomischen Struktur in der Nähe des prolabierenden Organs erstrecken,
1.2) ein Hilfswerkzeug (3) zur geführten Perforation der zu sezierenden anatomischen Region nahe des prolabierenden Organs, wobei das Hilfswerkzeug eine Stange (8), deren distaler Abschnitt (8a) umgebogen ist, der ein spitzes Ende (9) und ein Mittel (10) zur lösbaren Verbindung mit einem freien Verankerungsende (7) des Implantats (2) aufweist, sowie einen mit dem proximalen Abschnitt der Stange (8) fest verbundenen Griff (11) umfasst, wobei einerseits mindestens ein freies Verankerungsende (7) ein Zugelement, beispielsweise eine Schlaufe (12), aufweist und andererseits der distale Abschnitt (8a) des Perforationshilfswerkzeugs ein Element zum lösbaren Greifen, beispielsweise einen Haken (13), des Zugelements (12) aufweist,
**dadurch gekennzeichnet, dass** das Implantat (2) Mittel zum vorübergehenden septischen Schützen in Bezug auf die zu sezierende anatomische Region umfasst, wobei die Mittel zum vorübergehenden Schützen eine Tasche (19) umfassen, die aus mindestens einer in Bezug auf die zu sezierende anatomische Region isolierenden Wand besteht, wobei die besagte Tasche dazu eingerichtet ist, die Tragbasis (5) des Implantats und die Aufhängungsarme (61 bis 64) in einer gefalteten Position auf der Tragbasis aufzunehmen, wobei jedoch mindestens ein Zugelement (12) die Wand durchdringt und auf der Außenseite der Tasche (19) bleibt.

2. Einheit nach Anspruch 1, **dadurch gekennzeichnet, dass** der distale Abschnitt (8a) des Hilfswerkzeugs (3) zur geführten Perforation einen zugespitzten Abschnitt (14) aufweist, der zum Griff (11) hin durch einen durchgezogenen Abschnitt (15) verlängert ist, in dem mindestens eine Kerbe (16) zum Eindringen in und Zurückhalten eines Zugelements (12) des Implantats (2) vorgesehen ist.

3. Einheit nach Anspruch 1, **dadurch gekennzeichnet, dass** sie mindestens einen Zwischenzugstab (4) umfasst, der an einem Ende (4a) ein Zugelement, beispielsweise eine Schlaufe (17), und an seinem anderen Ende (4b) ein Element zum lösbaren Greifen, beispielsweise einen Haken (18), aufweist, wobei der Stab eine glatte Außenfläche für eine nicht-traumatische Durchführung in die zu sezierende anatomische Region hat, die mit dem Hilfswerkzeug (3) zur geführten Perforation perforiert wurde.

4. Einheit nach Anspruch 1, **dadurch gekennzeichnet, dass** die Wand aus einem thermoplastischen Material hergestellt ist, das von Öffnungen (20) aus, die in der Tasche (19) erzeugt wurden, durch den Zug eines sich entfaltenden und die besagten Öffnungen vergrößernden Aufhängungsarms (61 bis 64) zerreißbar und/oder teilbar ist.

5. Einheit nach Anspruch 1, **dadurch gekennzeichnet, dass** die Tasche eine Verschlusslasche (21) auf Höhe der Vorderkante (5a) der Basis (5) des Implantats aufweist.

6. Implantat (2) zur chirurgischen Behandlung eines Prolapses, der ein prolabierendes Organ oder einen Abschnitt eines prolabierenden Organs betrifft, das aus der Gruppe bestehend aus dem Rektum, den weiblichen Genitalorganen, darunter der Uterus und die Vagina, und der Blase ausgewählt ist, wobei das Implantat die Form eines dünnen und anpassbaren Teils hat und Folgendes umfasst:
- eine Basis (5) zum Tragen oder Stützen des prolabierenden Organs, die sich von einer Vorderkante (5a) zu einer Hinterkante (5b) erstreckt, mit gegebenenfalls zwei Seitenkanten (5c, 5d) zwischen der besagten Vorderkante und der besagten Hinterkante,
- mindestens vier Aufhängungsarme (61 bis 64), darunter zwei gegenüberliegende vordere (61, 62) und zwei gegenüberliegende hintere (63, 64), die sich jeweils von der Tragbasis (5) aus zu einem freien Verankerungsende (7) hin in einer stabilen anatomischen Struktur in der Nähe des prolabierenden Organs erstrecken,
- mindestens ein freies Verankerungsende (7), das ein Zugelement, beispielsweise eine Schlaufe (12), aufweist,
**dadurch gekennzeichnet, dass** es Mittel zum vorübergehenden Schützen umfasst, wobei die letzteren eine Tasche (19) umfassen, die aus mindestens einer in Bezug auf die zu sezierende anatomische Region isolierenden Wand besteht, wobei die besagte Tasche dazu eingerichtet ist, die Tragbasis (5) des Implantats und die Aufhängungsarme (61 bis 64) in einer gefalteten Position auf der Tragbasis aufzunehmen, wobei jedoch mindestens ein Zugelement (12) die Wand durchdringt und auf der Außenseite der Tasche (19) bleibt.

7. Implantat nach Anspruch 6, **dadurch gekennzeichnet, dass** die vier freien Verankerungsenden (7) jeweils vier Zugelemente (12) aufweisen.

8. Implantat nach Anspruch 6, **dadurch gekennzeichnet, dass** die Wand aus einem thermoplastischen Material hergestellt ist, das von Öffnungen (20) aus, die in der Tasche (19) erzeugt wurden, durch den Zug eines sich entfaltenden und die besagten Öffnungen vergrößernden Aufhängungsarms (61 bis 64) zerreißbar und/oder teilbar ist.

9. Implantat nach Anspruch 6, **dadurch gekennzeichnet, dass** die Tasche eine Verschlusslasche (21) auf Höhe der Vorderkante (5a) der Basis (5) des Implantats aufweist.

10. Implantat nach Anspruch 6, **dadurch gekennzeichnet, dass** das dünne und anpassbare Teil ein durchbrochenes prothetisches Gewirke, das aus einem einzigen Teil hergestellt ist, auf der Basis einer Anordnung umfasst, die aus mehreren Fadenscharen aus einem biokompatiblen Polymermaterial besteht und die kontinuierlich in Richtung des Gewirkes die Tragbasis (5) und die vier Aufhängungsarme (61 bis 64) auf beiden Seiten der Tragbasis (5) umfasst, und wobei die Elastizität des Gewirkes in der Tragbasis größer als die Elastizität des Gewirkes in jedem der Aufhängungsarme ist.

11. Implantat nach Anspruch 6, **dadurch gekennzeichnet, dass** die flächenbezogene Masse des Gewirkes in der Tragbasis (5) größer als die flächenbezogene Masse des Gewirkes in jedem der Aufhängungsarme (61 bis 64) ist.

12. Implantat nach Anspruch 10 oder 11, **dadurch gekennzeichnet, dass** das prothetische Gewirke Folgendes umfasst:
- mindestens zwei Basisscharen, eine vordere Schar und eine hintere Schar, die sich auf der Oberfläche der Tragbasis (5) und der vier Arme (61 bis 64) erstrecken, wobei die zwei besagten Basisscharen vermascht sind und eine erste Legung definieren,
- mindestens zwei Zusatzscharen hinter der besagten hinteren Basisschar, die sich hauptsächlich auf den jeweiligen Oberflächen der Aufhängungsarme (61 bis 64) erstrecken, wobei die zwei besagten Zusatzscharen unvermaschte Scharen von Teilschüssen ist und eine zweite Legung definieren.

13. Implantat nach Anspruch 6, **dadurch gekennzeichnet, dass** das dünne und anpassbare Teil ein Verbundstoff ist und Folgendes umfasst:
- einen porösen Textilträger, der eine Anordnung von Fäden umfasst, die sich jeweils aus mindestens einer Faser aus Polymermaterial zusammensetzen, wobei der besagte Textilträger sich auf der Tragbasis (5) und auf den Aufhängungsarmen (61 bis 64) und diesen entsprechend erstreckt,
- ein hydrophiles resorbierbares Material (30), das hauptsächlich die Tragbasis (5) bedeckt.

14. Implantat nach Anspruch 13, **dadurch gekennzeichnet, dass** der poröse Textilträger eine mikroporöse Struktur definiert, die die Zwischenräume zwischen mindestens zwei Fäden an den Kontaktstellen eines Fadens mit mindestens einem anderen Faden umfasst, und das hydrophile resorbierbare Material in der Tragbasis (5) den Textilträger beschichtet, wobei eine Schicht gebildet wird, die die Anordnung von Fäden umhüllt und in diese eindringt, wobei mindestens die mikroporöse Struktur abgedichtet wird, ohne jedoch eine ebene Lage zu bilden, die mindestens eine Fläche des Textilträger beschichtet.

## Claims

1. An assembly (1) for the surgical treatment of a prolapse affecting a protruding organ or portion of organ, selected from the group consisting of the rectum, the female genital organs, including uterus and vagina, and the bladder, comprising :
1.1) an implant (2) having the shape of a thin and comfortable part, and comprising :
- a base (5) for supporting or holding the protruding organ, extending from a front edge (5a) to a rear edge (5b), optionally with two side edges (5c, 5d) between said front and rear edges,
- at least four suspension arms, including two front arms (61, 62) facing each other, and two rear arms (63, 64) facing each other, each extending from the support base toward a free anchoring end (7) in a stable anatomical structure in the vicinity of the protruding organ
1.2) an ancillary (3) of guided puncturing of the anatomical region to be dissected, in the vicinity of the protruding organ, comprising a curved pin (8) which includes the distal portion (8a), and a sharp end (9), and a means (10) for detachable connection with a free anchoring end (7) of the implant (2), as well as a handle (11) integral with the proximal portion of the pin (8), on the one hand at least one free anchoring end (7) includes a pulling element, for example a loop (12), and on the other hand the distal portion (8a) of the puncturing ancillary includes a detachable gripping element, for example a hook (13), of the pulling element (12),
**characterized in that** the implant (2) comprises temporary protection means with regard to the septic anatomical region to be dissected, the temporary protection means comprising a pocket (19) consisting of at least one insulating wall with regard to the anatomical region to be dissected, said pocket being arranged to receive the support base (5) of the implant and the suspension arms (61 to 64) in the folded position on the support base, but with at least one pulling element (12) passing through the wall and remaining outside of the pocket (19).

2. The assembly according to claim 1, **characterized in that** the distal portion (8a) of the guided puncturing ancillary (3) includes a tapered portion (14) extending toward the handle (11) by a solid portion (15) in which there is arranged at least one notch (16) for the penetration and the retaining of a pulling element (12) of the implant (2).

3. The assembly according to claim 1, **characterized in that** it comprises at least one intermediate pulling rod (4), including, at one end (4a) a pulling element, for example a loop (17), and at its other end (4b) a detachable gripping element, for example a hook (18), the rod having a smooth outer surface for a non-traumatic passage in the anatomical region to be dissected, punctured with the guided puncturing ancillary (3).

4. The assembly according to claim 1, **characterized in that** the wall is made of a thermoplastic material tearable and/or cuttable, from openings (20) generated in the pocket (19) by the pulling of a suspension arm (61 to 64) which unfolds, and enlarges said openings.

5. The assembly according to claim 1, **characterized in that** the pocket includes a closure flap (21) at the front edge (5a) of the base (5) of the implant.

6. An implant (2) for the surgical treatment of a prolapse affecting a protruding organ or portion of organ selected from the group consisting of the rectum, the female genital organs, including uterus and vagina, and the bladder, said implant having the shape of a thin and comfortable part, and comprising :
- a base (5) for supporting or holding the protruding organ, extending from a front edge (5a) to a rear edge (5b) optionally with two side edges (5c, 5d) between said front and rear edges,
- at least four suspension arms (61 to 64), including two front arms (61, 62) facing each other, and two rear arms (63, 64) facing each other, each extending from the support base (5) toward a free anchoring end (7) in a stable anatomical structure in the vicinity of the protruding organ,
- at least one free anchoring end (7) including a pulling element, for example a loop (12),
**characterized in that** it comprises temporary protection means, these comprising a pocket (19) consisting of at least one insulating wall with regard to the anatomical region to be dissected, said pocket being arranged to receive the support base (5) of the implant and the suspension arms (61 to 64) in the folded position on the support base, but with at least one pulling element (12) passing through the wall and remaining outside of the pocket (19).

7. The implant according to claim 6, **characterized in that** the four free anchoring ends (7) respectively include four pulling elements (12).

8. The implant according to claim 6, **characterized in that** the wall is made of a thermoplastic material tearable and/or cuttable, from openings (20) generated in the pocket (19) by pulling a suspension arm (61 to 64) which unfolds, and enlarges said openings.

9. The implant according to claim 6, **characterized in that** the pocket includes a closure flap (21) at the front edge (5a) of the base (5) of the implant.

10. The implant according to claim 6, **characterized in that** the thin and comfortable part comprises a one-piece openwork prosthetic knit fabric, based on an arrangement consisting of several sets of yarns made of a biocompatible polymer material, comprising, in a continuous manner along the direction of the knit fabric, the support base (5), and the four suspension arms (61 to 64) on either side of the support base (5), and the elasticity of the knit fabric in the support base being greater than the elasticity of the knit fabric in each of the suspension arms.

11. The implant according to claim 6, **characterized in that** the mass per unit area of the knit fabric in the support base (5) is greater than the mass per unit area of the knit fabric in each of the suspension arms (61 to 64).

12. The implant according to claim 10 or 11, **characterized in that** the prosthetic knit fabric comprises :
- at least two basic sets, a front set and a rear set, extending over the surface of the support base (5) and the four arms (61 to 64), said two basic sets being meshing sets and defining a first weave,
- at least two additional sets, at the back of said rear basic set, extending mainly over the respective surfaces of the suspension arms (61 to 64), said two additional sets being partial-frame unmeshing sets and defining a second weave.

13. The implant according to claim 6, **characterized in that** the thin and comfortable part is composite, and comprises :
- a porous textile support comprising an arrangement of yarns each composed of at least one strand made of a polymer material, said textile support extending over and corresponding to the support base (5) and the suspension arms (61 to 64),
- a hydrophilic resorbable material (30) coating mainly the support base (5).

14. The implant according to claim 13, **characterized in that** the porous textile support defines a microporous texture comprising the gaps comprised between at least two yarns at the contact locations of a yarn with at least one other yarn, and, in the support base (5), the hydrophilic resorbable material coats the textile support, by forming a film wrapping and penetrating the arrangement of yarns, by occluding at least the microporous texture, but without forming a planar layer that coats at least one textile support face.
